# EUROPEAN PATENT SPECIFICATION

(11) **EP 3 298 404 B1**
(45) Date of publication and mention of the grant of the patent: **20.04.2022**
(21) Application number: 16725437.4
(22) Date of filing: 18.05.2016
(51) Int. Cl.: A23L 33/00, A23C 9/20, G01N 33/53

(54) **KIT-OF-PARTS TO IDENTIFY MOTHER'S MILK MISSING FUCOSYLTRANSFERASE-2 DEPENDENT GLYCANS**
TEILESATZ ZUR IDENTIFIZIERUNG VON FEHLENDEN FUCOSYLTRANSFERASE-2-ABHÄNGIGEN GLYCANEN IN MUTTERMILCH
ENSEMBLES DE PIÈCES POUR IDENTIFIER LES GLYCANES DÉPENDANT DES FUCOSYLTRANSFÉRASES-2 ABSENTES DU LAIT MATERNEL

(30) Priority: 19.05.2015 EP 15168137
(43) Date of publication of application: 28.03.2018
(73) Proprietor: Société des Produits Nestlé S.A., 1800 Vevey (CH)
(72) Inventor: BRASSART, Dominique, 1022 Chavannes-Près-Renens (CH); SPRENGER, Norbert, 1073 Savigny (CH)
(74) Representative: Gagliardi, Tatiana
(86) International application number: PCT/EP2016/061066
(87) International publication number: WO 2016/184880

(56) References cited:
- WO-A1-2009/136859
- WO-A1-2012/069416
- WO-A2-2008/105764
- CN-A- 1 237 706
- PERRET S ET AL: "Structural basis for the interaction between human milk oligosaccharides and the bacterial lectin PA-IIL of Pseudomonas aeruginosa", BIOCHEMICAL JOURNAL, PORTLAND PRESS LTD, GB, vol. 389, 24 March 2005 (2005-03-24), pages 325-332, XP002417556, ISSN: 0264-6021, DOI: 10.1042/BJ20050079

## Description

### Field of the invention

The invention relates to a matrix and a diagnostic kit comprising a matrix to identify a deficiency of 2'fucosyl-glycans in mother's milk fucosyltransferase-2 (FUT2) dependent glycans. Furthermore, the invention relates to a kit-of-parts comprising such a diagnostic kit and 2'fucosyl-glycans.

### Background of the invention

Human milk contains a variety of distinct oligosaccharides from which most are fucosylated see for instance Newburg, J Anim Sci, 2009;87 (Suppl 1);26-34. However, different women synthesize different subsets of oligosaccharides, and the total amount and relative abundance of human milk oligosaccharides changes during the course of lactation as well.

The most extreme interpersonal variation is found with respect to human milk oligosaccharide fucolysation and is based on women's Secretor and Lewis group status, which is genetically determined.

The majority of women express fucosyltransferase II (FUT2) that connects fucose to the terminal galactose by an α-1,2-linkage. In addition, some women express fucosyltransferase III (FUT3) which connects fucose to N-acetyl-glucosamine by an α-1,4-linkage. Women who express both FUT2 and FUT3 are called Lewis-positive secretors; women who express FUT2 only are called Lewis-negative Secretors; women who express FUT3 only are called Lewis-positive non-Secretors; the small group of women who express neither FUT2 nor FUT 3 are called Lewis-negative non Secretors. Summarized: women who are non-Secretors do not produce α-1,2- linked fucosylated oligosaccharides, 20-25% of all European women (Newburg et al, Glycobiol, 2004;14(3);253-263).

As many enteropathogens as for instance Campylobacter species, which cause diarrhoea bind to 2'-fucosylated glycans said glycans seem to have an important role when provided to an infant as they seem to bind to the enteropathogens. In this way these pathogens are prevented from attaching the surface of the intestinal mucosa. It has been demonstrated indeed that breastfed infants receiving human milk with a high total amount of α-1,2-linked oligosaccharides were better protected against diarrhoea than breasted infants receiving milk with a low total amount of α-1,2-linked oligosaccharides (Newburg, *J Anim Sci,* see above).

A similar teaching can be found in Newburg et al, Glycobiol, see above, where the ratio between alpha-1,2-linked fucosylated oligosaccharides and non-1,2-linked fucosylated oligosaccharides in milk from Secretor mothers (Le(a-/b+) and Le(a-/b-)) was correlated with the frequency of diarrhoea in their breastfed infants. The authors concluded that there was a clear relationship between the content of alpha-1,2-linked oligosaccharides in the mother's milk and the infants' risk of obtaining diarrhoea.

WO2011/136648 from Nutricia is focused on the use of a composition comprising human milk oligosaccharides (HMOs) such as 2-fucosylated oligosaccharides for the preparation of a nutritional composition for feeding an infant, said infant being a Secretor and describes a method to supplement milk from a non-Secretor mother with 2-fucolysated oligosaccharides as well.

However this patent application does not refer to the advantageous prevention and/or treatment of URT infections in an infant or young child as described in the non-prepublished patent application PCT/EP14/074555 (now published as WO2015/071389) entitled: Compositions for use in the prevention or treatment of URT infections in infants or young children at risk; or to the advantageous prevention and/or treatment of allergies in an infant or young child as described in the non-prepublished patent application PCT/EP14/073623 (WO2015/071131) entitled: Compositions for preventing or treating allergies in infant from or fed by non-secretor mothers by providing fucosylated-oligosaccharides in particular among infants at risk or born by C-section.

In this last patent application it has been shown as well that the level of 2FL varies greatly in various human breast milk from various mothers. It shows a correlation between the amount of 2FL in the human breast milk fed to the infants during the first months of life and the risk of allergies (occurrence of Eczema and atopic diseases at 2 years). This demonstrates that the non Secretor status of a mother is not the only factor predictive of allergy risk but also that the amount of fucosylated oligosaccharide in the human breast milk is another factor. The lower the level of fucosylated oligosaccharide (e.g. 2FL) in the human breast milk fed to the infants in the first months of life, the higher the risk of allergy is (especially atopic diseases such as atopic dermatitis or eczema).

As the amount of 2'-fucosylated glycans in mother's milk may vary a lot therefore and this may have a great impact on the health of the infant there is apparently a need to measure this amount regularly. Identification and quantification could be performed by HPAEC analysis as has been described in WO 2012/069416. However, this is not an easy and cheap analysis which could be performed regularly and at home as well.

Furthermore it would be desirable to have doses of 2'fucosyl-glycans at hand to complete mother's milk with a deficiency of these compounds as well.

Patent applications WO 2008/105764 A2 and CN 1 237 706 A disclose matrices to detect analytes in breast milk in a test strip format, but no fucosyl-transferase-2 dependent glycans. Patent application WO 2009/136859 A1 discloses a matrix to detect mother's milk FUT2-dependent glycans comprising a glycan binding protein such as a lectin suitable to bind 2' fucosyl-glycans (the *Aleuria aurantia* lectin) by affinity chromatography. Perret *et al.* disclose a matrix to detect mother's milk FUT2-dependent glycans comprising a glycan binding protein such as a lectin suitable to bind 2' fucosyl-glycans (the fucose-binding lectin PA-IIL isolated from *P. aeruginosa* cytoplasm) in an enzyme-linked lectin assay (ELLA) format (Perret S et al.: "Structural basis for the interaction between human milk oligosaccharides and the bacterial lectin PA-IIL of Pseudomonas aeruginosa", Biochem. J. (2005) 389(2), 325-332).

### Summary of the invention

The present invention has for its object to provide a matrix as stated in the preamble comprising a line of a glycan binding protein such as a lectin or antibody suitable to bind 2'fucosyl-glycans and a line of a positive control to bind a mother's milk protein. According to an embodiment said matrix is porous and allows for transport by capillary force of compounds of mother's milk such as glycoproteins, a line of a glycan binding protein such as a lectin or antibody suitable to bind 2'fucosyl-glycans and a line of a positive control to bind a mother's milk protein. Especially, the glycan binding protein is the plant lectin from Ulex europaeus. More especially, the positive control comprises an antibody or a fragment thereof specific for a specific milk protein like lactalbumin. Both the glycan binding protein and the positive control have preferably been labelled with different dyes.

According to a further embodiment of the invention a diagnostic kit comprising one or more matrices according to the invention and a device suitable to receive such a matrix provided with a milk application window and a read-out window.

According to still a further embodiment of the invention a kit-of-parts comprising a diagnostic kit according to the invention and one or more feeding doses of 2'fucosyl-glycans such as of e.g. 100 - 5000 mg, e.g. 1000 - 5000 mg, e.g. 2000 - 5000 mg. As will be understood, these amounts may vary dependent on the age and the weight of the infant and young child. Preferably, the kit-of-parts comprises a diagnostic kit wherein the 2'fucosyl-glycans comprise 2'-fucosyllactose.

The kit-of-parts of the invention is for use both to identify a deficiency of 2'fucosyl-glycans in mother's milk and to supplement this deficiency. Preferably the kit-of-parts of the invention is for use to supplement the 2'fucosyl-glycans to a daily dose such as of e.g.100 mg - 5000 mg, e.g. 1000 - 5000 mg, e.g. 2000 - 5000 mg, 3000 - 5000 mg, 4000 - 5000 mg. As will be understood, these amounts may vary dependent on the age and the weight of the infant and young child.

### Figures

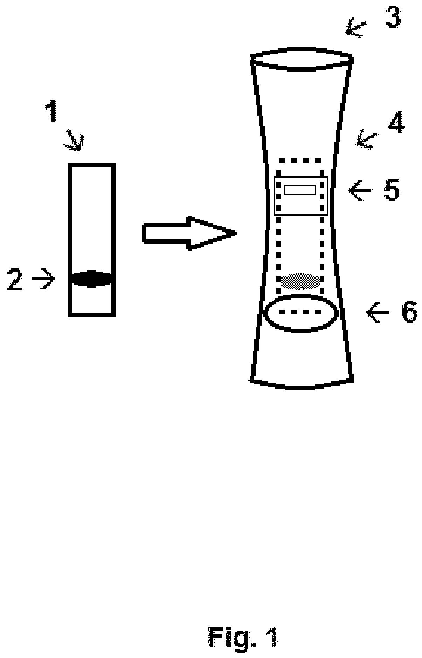

Figure 1 represents a diagnostic kit comprising
a matrix (1) with a line of plant lectin from Ulex europaeus and an antibody specific for lactalbumin and
a plastic device (3) in the form of a stick comprising a milk application window (6) and a read-out window (5). The matrix (1) is integrated in the device indicated with the dotted line (4).

### Detailed description of the invention

As used herein, the following terms have the following meanings.

The terms **'2'fucosyl-glycans'** or **'2-fucosylated oligosaccharides'** or **'alpha-1,2-linked fucosylated oligosaccharides'** have interchangeable been used and have all the same meaning: HMO's comprising a 2' fucosyl-epitope.

The term **"HMO"** or **"HMOs"** refers to human milk oligosaccharide(s). These carbohydrates are highly resistant to enzymatic hydrolysis, indicating that they may display essential functions not directly related to their caloric value. It has especially been illustrated that they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy terminal positions at the non-reducing ends. The HMOs can be acidic (e.g. charged sialic acid containing oligosaccharide) or neutral (e.g. fucosylated oligosaccharide).

A **"fucosylated oligosaccharide"** is an oligosaccharide having a fucose residue. It has a neutral nature. Some examples are 2-FL (2'-fucosyllactose), 3-FL (3-fucosyllactose), difucosyllactose, lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof. Without wishing to be bound by theory it is believed that the fucosyl- epitope of the fucosylated oligosaccharides may act as decoy at the mucosal surface. By a competition effect, it may prevent and/or limit the action of the pathogens responsible of infections (of viral or bacterial origin) or of their secreted components (e.g. toxins), especially by avoiding their binding to natural ligands, and this will therefore reduce the risk of infections, and particularly of URT infections or allergies.

The expressions **"fucosylated oligosaccharides comprising a 2'-fucosyl-epitope"** and **"2-fucosylated oligosaccharides"** encompass fucosylated oligosaccharides with a certain homology of form since they contain a 2'-fucosyl-epitope, therefore a certain homology of function can be expected. Without wishing to be bound by theory the 2'-fucosyl-epitope of these fucosylated oligosaccharides is believed to be particularly specific to pathogens (or their secreted components) involved in URT infections or allergies. Some examples are 2-FL (2' fucosyllactose), difucosyllactose, lacto-N-fucopentaose, lacto-N-fucohexaose, lacto-N-difucohexaose, fucosyllacto-N-hexaose, fucosyllacto-N-neohexaose, difucosyllacto-N-hexaose difuco-lacto-N-neohexaose, difucosyllacto-N-neohexaose, fucosyl-para-Lacto-N-hexaose and any combination thereof.

An example of lacto-N-fucopentaose is lacto-N-fucopentaose I.

The term **"infant"** means a child under the age of 12 months.

The expression **"young child"** means a child aged between one and three years, also called toddler.

The expressions **"to prevent URT infections", "preventing URT infections"** or **"prevention of URT infections"** mean avoiding that URT infections occur and/or decreasing the incidence of the URT infections (reduction of the frequency, i.e. the number of URT infections). The prevention of URT infections is during the treatment (i.e. during the administration of the composition of the present invention). It can also encompass the prevention of URT infections later in life. The term "later in life" encompasses the effect after the termination of the intervention or treatment. The effect "later in life" can be from 1 week to several months, for example from 2 to 4 weeks, from 2 to 6 weeks, from 2 to 8 weeks, from 1 to 6 months or from 2 to 12 months.

The expressions "to treat **URT infections", "treating URT infections"** or **"treatment of URT infections"** should be understood as comprising the decrease of the duration of the URT infections (number of days/weeks/years the infants or young children will suffer from URT infections), of the severity of URT infections (the consequences and/or the seriousness of URT infections). These expressions also encompass the relieve of the symptoms such as cough, sore throat, runny nose, nasal congestion, headache, low grade fever, facial pressure, sneezing, and combinations thereof, and/or the decrease of complications caused by URT infections on the infant or young child health, such as otitis media and sometimes bronchitis, and/or the decrease of pain, and/or the decrease of tiredness, and/or the ease of the sleep and/or the stabilization of the activity of the infants or young children suffering from URT infections.

The **"mother's milk"** should be understood as the breast milk or the colostrum of the mother.

By **"a deficiency of 2'fucosyl-glycans' in mother's milk"** or **"mother's milk missing fucosyltransferase-2 (FUT2) dependent glycans"** it is meant a mother's milk that lacks, is depleted of or is poor in 2-fucosylglycans.The milk may contain no (or almost no) fucosylated oligosaccharides, or a low amount of fucosylated oligosaccharides such as an amount which represents less than 50%, or less than 40%, or less than 35%, or less than 30%, or less than 25%, or less than 20%, or less than 15%, or less than 10%, or less than 5%, or less than 3%, or less than 2% of the mean (or average) amount that is generally found in the mother's milk or
mother's milk fed to the infant or young child is deficient in at least one 2-fucosylated oligosaccharide(s) in the sense that it has a concentration of said 2-fucosylated oligosaccharide(s) of less than 3000 mg/L, less than 2000 g/L, less than 1000mg/l, less than 500 mg/L, less than 300 mg/L, less than 100 mg/L, less than 50 mg/L, less than 10 mg/L, less than 1 mg/L.

The 2'fucosylated-oligosaccharide to be used in the feeding dose may be isolated by chromatography or filtration technology from a natural source such as animal milks. Alternatively, it may be produced by biotechnological means using specific 2'fucosyltransferases either through the use of enzyme-based fermentation technology (recombinant or natural enzymes) or microbial fermentation technology. In the latter case, microbes may either express their natural enzymes and substrates or may be engineered to produce respective substrates and enzymes. Single microbial cultures and/or mixed cultures may be used. Fucosylated oligosaccharide formation can be initiated by acceptor substrates starting from any degree of polymerization (DP), from DP = 1 onwards. Alternatively, 2'fucosylated oligosaccharides may be produced by chemical synthesis from lactose and free fucose. Fucosylated oligosaccharides are also available for example from Kyowa, Hakko, Kogyo of Japan. Alternatively, fucosyl-glycans might be produced by employing the reverse reaction of fucosylhydrolases and suitable fucose donor substrates such as fucose linked to a carrier.

All percentages are by weight unless otherwise stated.

The invention will now be described in further details. It is noted that the various aspects, features, examples and embodiments described in the present application may be compatible and/or combined together.

In addition, in the context of the invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

A first object of the invention is to provide a diagnostic kit to analyse the presence or absence and/or the amount of 2'fucosyl-glycans in mother's milk in an easy and convenient way which can be applied at home as well.

In a first embodiment this diagnostic kit comprises
- one or more matrices which allow the transport of compounds of mother's milk by capillary force, for instance made of silica, a line of a plant lectin from Ulex europaeus suitable to bind 2'fucosyl-glycans and a line of a positive control to bind a mother's milk protein, both labelled with different dyes and
- a device suitable to receiving a matrix with a milk application window and a read out window to analyse,
   where the device may be used several times by receiving various matrices.

With one type of matrix, the application of a milk sample to the application window will lead to migration of small milk components such as fucosyl-glycans. When reaching the lectin and antibody lines these will migrate with the milk. Lectins and antibodies that bound their respective substrate (fucosyl-glycans or specific milk protein) will migrate less and reach the height of the read-out window. Appearance of two bands indicates that the test worked (positive control) and that 2'fucosyl-glycans are present. If no 2'fucosyl-glycans are present the second band will migrate outside the read-out window and not be seen.

If two bands appear the intensity of the band will indicate the relative amount of 2'fucosyl-glycans. A scale such as from 1 to 4 corresponding to band intensity seen on the read-out window will be provided on the device. The scale will allow to select the optimal supplementation dose of the provided 2'fucosyl-glcans.

In an alternative embodiment of the invention the matrix will have the glycan binding protein as lectin and antibody positioned in such a way that application of milk will lead to appearance or quenching of colour without migration of the lectin or antibody. In this embodiment preferably labelling of the glycan binding protein and antibody with fluorescent dyes as well as a light source should be provided to read the colour appearance and quenching on the device. The intensity of the colour will indicate the relative amount of 2'fucosyl-glycans.

The principle of the application of the diagnostic kit may be compared with other generally known immune tests.

A second object of the invention is to provide a kit-of-parts to analyse the content of 2'fucosyl-glycans in mother's milk in a convenient, easy and cheap way, and to provide feeding doses of said glycans to supplement an eventual deficiency.

This kit-of-parts according to the present invention comprises:
- a diagnostic kit as disclosed above
- one or more feeding doses with 2'fucosyl-glycans to supplement the feeding with the mother's milk if necessary.

If the line coloured with the dye labelled to the 2'fucosyl-glycans did not move while the control one did as exemplified in figure 1, the mother's milk lacks 2'fucosyl-glycans.

In that case one or more feeding doses of 2'fucosyl-glycans should be given to the infant or young child fed with the mother's milk in such an amount that an amount of 0,1 g - 5 g per day, especially 2 - 5 g / day be achieved.

Furthermore the line coloured with the dye labelled to the 2'fucosyl-glycans does not indicate only that 2'fucosyl-glycan is present but the intensity of the colour of the quenching is related to the amount of 2'fucosyl-glycans as well.

This feeding with extra 2'fucosyl-glycans may take place by offering the feeding dose or feeding doses in several ways as for instance:
dissolved in water or a small amount of breast milk to allow for application of a spoon or a syringe preferably during the normal breast feeding session, in an amount to have at least the minimum amount as indicated above given to the infant or young child.
However, the feeding dose of 2'fucosyl-glycans may be given added to other nutrition compositions as fruit or vegetables as well.

As indicated, the device may repeatedly be used to receiving a new matrix each time to read out the amount of 2'fucosyl-glycans present in the mother's milk.

### Example

Figure 1 shows a diagnostic kit according to the invention comprising a rectangular matrix (1) with a line of plant lectin from Ulex europaeus and a line of an antibody specific for lactalbumin, both bound to a dye. This matrix may be put into the milk with the side where the lines are. Then the matrix should be put into the transparent device (3) at position (4) with the side where the lines are at the height of the milk application window (6). With the read-out window (5) the position of the lines and therewith the presence of the 2'fucosyl-glycans should be determined provided that the position of the control line denoting lactalbumin is found on the expected place.

## Claims

1. Matrix to identify *in vitro* mother's milk missing fucosyltransferase-2 (FUT2) dependent glycans comprising a line of a glycan binding protein such as a lectin or antibody suitable to bind 2'fucosyl-glycans and a line of a positive control to bind a mother's milk protein.

2. Matrix according to claim 1 which is porous and allows for transport by capillary force of compounds of mother's milk such as glycoproteins.

3. Matrix according to claim 1 or 2 wherein the glycan binding protein is the plant lectin from Ulex europaeus.

4. Matrix according to any of the preceding claims wherein the positive control comprises an antibody or a fragment thereof specific for a specific milk protein like lactalbumin.

5. Matrix according to any of the preceding claims wherein both the glycan binding protein and the positive control have been labelled with different dyes such as fluorescent dyes.

6. Diagnostic kit comprising one or more matrices according to any of the preceding claims and a device suitable to receive such a matrix provided with a milk application window and a read-out window.

7. Diagnostic kit according to claim 6 wherein the intensity of the band or of the colour seen on the read-out window indicates the relative amount of 2'fucosyl-glycans.

8. Kit-of-parts comprising a diagnostic kit according to claim 6 or 7 and one or more feeding doses of 2'fucosyl-glycans such as of 100 - 5000 mg.

9. Kit-of-parts according to claim 8 wherein the 2'fucosyl-glycans comprise 2'-fucosyllactose.

10. Kit-of-parts as defined in claim 8 or 9 for use both to identify a deficiency of 2'fucosyl-glycans in mother's milk and to supplement this deficiency.

11. Kit-of-parts according to claim 10 to supplement the 2'fucosyl-glycans to a daily dose such as of 100 - 5000 mg.

## Patentansprüche

1. Matrix zur Identifizierung in vitro von Muttermilch, der fucosyltransferase-2 (FUT2)-abhängige Glycane fehlen, umfassend eine Reihe eines Glycanbindungsproteins wie ein Lectin oder einen Antikörper, die dazu geeignet sind, 2'Fucosylglycane zu binden, und eine Reihe einer Positivkontrolle, um ein Protein der Muttermilch zu binden.

2. Matrix nach Anspruch 1, die porös ist und den Transport durch Kapillarkraft von Verbindungen der Muttermilch, wie Glycoproteine, ermöglicht.

3. Matrix nach Anspruch 1 oder 2, wobei das Glycanbindungsprotein das Pflanzenlektin aus Ulex europaeus ist.

4. Matrix nach einem der vorstehenden Ansprüche, wobei die Positivkontrolle einen Antikörper oder ein Fragment davon umfasst, der bzw. das spezifisch für ein spezifisches Milchprotein, wie Lactalbumin, ist.

5. Matrix nach einem der vorstehenden Ansprüche, wobei sowohl das Glycanbindungsprotein als auch die Positivkontrolle mit unterschiedlichen Farbstoffen, wie Fluoreszenzfarbstoffen, markiert wurden.

6. Diagnostiksatz, umfassend eine oder mehrere Matrizen nach einem der vorstehenden Ansprüche und eine Vorrichtung, die zum Empfangen einer solchen Matrix geeignet ist, die mit einem Milchanwendungsfenster und einem Auslesefenster bereitgestellt wird.

7. Diagnostiksatz nach Anspruch 6, wobei die Intensität des Bandes oder der Farbe, die auf dem Auslesefenster zu sehen ist, die relative Menge von 2'Fucosylglycane angibt.

8. Teilesatz, umfassend ein Diagnostiksatz nach Anspruch 6 oder 7 und eine oder mehrere Zugabedosen von 2'Fucosylglycanen, wie 100 - 5000 mg.

9. Teilesatz nach Anspruch 8, wobei die 2'Fucosylglycane 2'-Fucosyllactose umfassen.

10. Teilesatz nach Anspruch 8 oder 9, beide zur Verwendung zum Identifizieren eines Mangels an 2'Fucosylglycanen in der Muttermilch und zum Ergänzen dieses Mangels.

11. Teilesatz nach Anspruch 10, um die 2'Fucosylglycane zu einer Tagesdosis, wie von 100 - 5000 mg, zu ergänzen.

## Revendications

1. Matrice pour identifier *in vitro* des glycanes dépendants de fucosyltransférase-2 (FUT2) absents du lait maternel comprenant une lignée d'une protéine de liaison au glycane telle qu'une lectine ou un anticorps appropriée pour se lier à des 2'-fucosyle-glycanes et une lignée d'un témoin positif pour se lier à une protéine du lait maternel.

2. Matrice selon la revendication 1 qui est poreuse et permet le transport par force capillaire de composés du lait maternel tels que des glycoprotéines.

3. Matrice selon la revendication 1 ou 2 dans laquelle la protéine de liaison au glycane est la lectine de plante extraite d'Ulex europaeus.

4. Matrice selon l'une quelconque des revendications précédentes dans laquelle le témoin positif comprend un anticorps ou un fragment de celui-ci spécifique pour une protéine de lait spécifique comme la lactalbumine.

5. Matrice selon l'une quelconque des revendications précédentes dans laquelle à la fois la protéine de liaison au glycane et le témoin positif ont été marqués avec différents colorants tels que des colorants fluorescents.

6. Kit de diagnostic comprenant une ou plusieurs matrices selon l'une quelconque des revendications précédentes et un dispositif approprié pour recevoir une telle matrice pourvu d'une fenêtre d'application de lait et d'une fenêtre de lecture.

7. Kit de diagnostic selon la revendication 6 dans lequel l'intensité de la bande ou de la couleur vue sur la fenêtre de lecture indique la quantité relative de 2'-fucosyle-glycanes.

8. Kit de pièces comprenant un kit de diagnostic selon la revendication 6 ou 7 et une ou plusieurs doses d'alimentation de 2'-fucosyle-glycanes telles que de 100 à 5 000 mg.

9. Kit de pièces selon la revendication 8 dans lequel les 2'-fucosyle-glycanes comprennent du 2'-fucosyllactose.

10. Kit de pièces tel que défini dans la revendication 8 ou 9 destiné à être utilisé à la fois pour identifier un déficit en 2'-fucosyle-glycanes dans le lait maternel et pour combler ce déficit.

11. Kit de pièces selon la revendication 10 pour compléter les 2'-fucosyle-glycanes à une dose quotidienne telle que de 100 à 5 000 mg.
